(19) Europäisches Patentamt
European Patent Office
Office européen des brevets

(11) Publication number: 0 237 450 B1

# (12) EUROPEAN PATENT SPECIFICATION

(45) Date of publication of patent specification: 15.05.91 Bulletin 91/20

(51) Int. Cl.$^5$: C07F 15/00, A61K 31/28

(21) Application number: 87420061.1

(22) Date of filing: 04.03.87

## (54) Platinum (IV) complexes.

(30) Priority: 07.03.86 JP 48625/86

(43) Date of publication of application: 16.09.87 Bulletin 87/38

(45) Publication of the grant of the patent: 15.05.91 Bulletin 91/20

(84) Designated Contracting States: DE FR GB

(56) References cited:
EP-A- 0 154 589
GB-A- 2 148 891
US-A- 4 550 187
J. CLIN. HEMATOL. ONCOL., vol. 7, no. 1, 1977, pages 231-241; L.M. HALL et al.: "Analogs of sulfato 1,2-diaminocyclohexane platinum (II)(SHP) II. Modifications other than leaving ligand"

(56) References cited:
JOURNAL OF MEDICINAL CHEMISTRY, vol. 21, no. 12, 1978, pages 1315-1318, American Chemical Society, Washington, US; Y. KIDANI et al.: "Antitumor activity of 1,2-diaminocyclohexane-platinum complexes against sarcoma-180 ascites form"

(73) Proprietor: Kidani, Yoshinori
2-718, Mataho Kodan-jutaku 2-1, Mataho-cho
Nishi-ku Nagoya-shi Aichi-ken (JP)

(72) Inventor: Kidani, Yoshinori
Mataho Kohdan Jutaku 2-718 1, Mataho-cho
2-chome
Nishi-ku Nagoya-shi Aichi-ken (JP)
Inventor: Noji, Masahide
184-5, Aza Fukazawa Ohaza Kitsuko
Moriyama-ku
Nagoya-shi Aichi-ken (JP)

(74) Representative: Maureau, Pierre et al
Cabinet GERMAIN & MAUREAU Le Britannia - Tour C 20, Boulevard E. Déruelle
F-69003 Lyon (FR)

EP 0 237 450 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to new platinum(IV) complexes which exhibit antineoplastic or antitumor activity as demonstrated by the test against mousse leukemia L-1210. The platinum(IV) complexes of this invention contains a 1,2-cyclohexanediamine (abbreviated as dach) or 2-(aminomethyl)-cyclohexylamine (abbreviated as amcha) as a ligand.

In recent years, a number of platinum(II) complexes, including well-known cisplatin, have been synthetized and have been reported to have antitumor activity. We the present inventors, have synthetized some platinum(II) complexes, as disclosed, for example, in documents JA-A-31648/78, JA-A-35013/80, JA-A-130992/80, JA-A-103192/81, JA-A-156416/82, JA-A-16895/82, JA-A-21697/84, JA-A-34982/85, JA-A-349883/85, JA-A-156416/82, JA-A-109521/85, JA-B-29957/83 and JA-B-196887/85, as well as documents US-C-4,169,846 ; US-C-4,200,583 ; US-C-4,256,652 ; US-C-4,255,347 ; US-C-4,551,524 and documents EP-C-1126 and EP-C-8936, European Patent Appln.Nos. 83 303659.3 and 84 305304.2.

We have also synthetized some organoplatinum(IV) complexes, as disclosed, for example, in documents JA-A-87295/85 and JA-A-109521/85.

The document US-C-4 550 187 has also described a method for preparing various stereoisomers of platinum(IV) antineoplastic agents, such as (1S, 2S) – tetrachloro-trans-dach-platinum(IV), (1R, 2R) – tetrachloro-trans-dach-platinum(IV), and tetrachloro-cis-dach-platinum(IV).

the following documents, i.e. :

– J. Clin. Hematol. Oncol, vol. 7, n°1, 1977, pages 231-241 ; L.M HALL et al, entitled "Analogs of sulfato 1,2 diaminocyclohexane platinum (II) (SHP) II : modifications other than leaving ligand" has described for antitumor activity cis-dichloro-trans-dihydroxo-dach platinum(IV), cis-dichloro-trans-dinitro-dach-platinum(IV), cis-malonato-trans-dihydroxo-dach-platinum(IV), and cis-malonato-trans-dinitro-dach platinum(IV).

The document EP-A-0 154 589 has also described pure stereoisomers of tetrahalo-dach-platinum(IV), e.g of the types trans-d, trans-ℓ, trans-d,ℓ, and cis, all these isomerically pure compounds being described as active against leukemia.

Finally, the document GB-A-2 148 891 has described various platinum-diamine complexes, such as trans-dihydroxo-cis-oxalato-amcha-platinum(IV), cis-dichloro-trans-dihydroxo-amcha-platinum(IV), cis-tetrachloro-amcha-platinum(IV) as having a carcinostatic effect.

The compounds disclosed in the last three documents appear to have some toxicity. It is thus the purpose of this invention to obtain new amcha-platinum(IV) or dach-platinum(IV) complexes, exhibiting less toxicity while keeping a substantial antineoplastic or antitumor activity.

Now we have succeeded in synthetizing new organoplatinum(IV) compounds having anyone of the following chemical formulae, and having for each chemical structure different stereoisomers, in pure form or admixture.

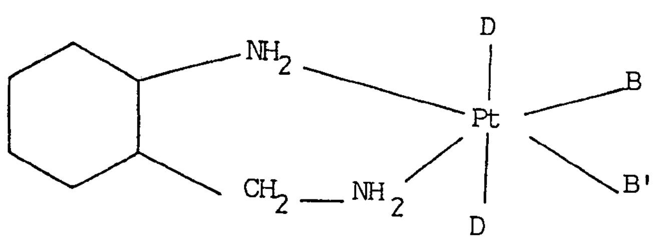

wherein B and B′ taken together denote a group of the formula

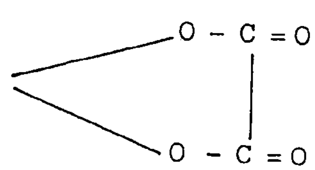

or a group of the formula

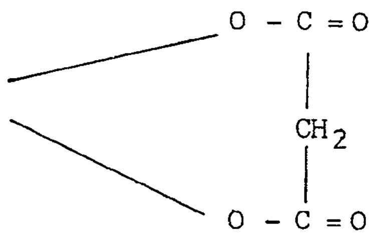

or B and B′ each denote a chloro group, and D denotes a chloro group, a nitrato group, or a hydroxo group

The amcha ligand may show a configuration selected from cis-ℓ-, cis-d-, trans-ℓ- and trans-d-.

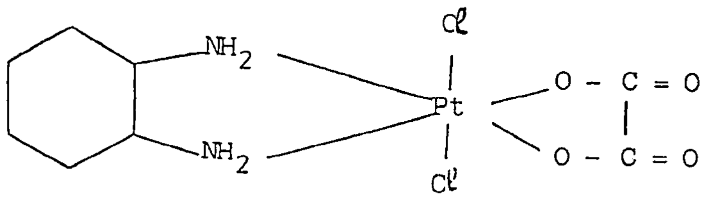

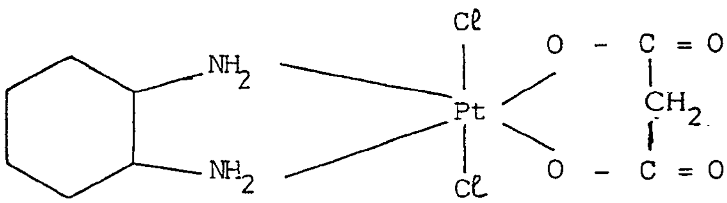

In formulae (2) and (3), the dach ligand may adopt three stereo-isomers according to the following steric structure :

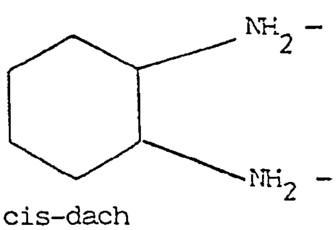

cis-dach
(1R,1S)

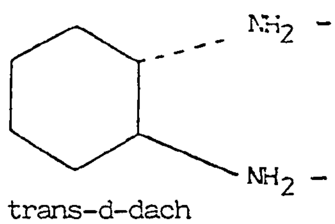

trans-d-dach
(1S,2S)

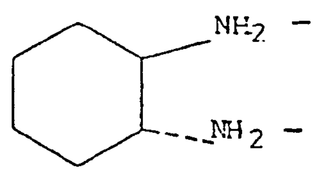

trans-ℓ -dach
(1R,2R)

In formula (1), the amcha ligand may adopt 4 stereo-isomers according to the following steric structure :

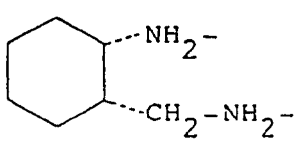

cis-d-amcha
(1S, 2S)

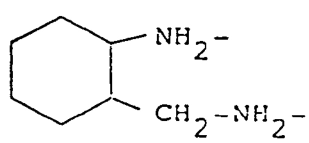

cis-ℓ-amcha
(1R, 2R)

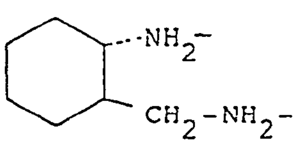

trans-d-amcha
(1S, 2R)

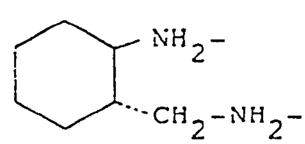

trans-ℓ-amcha
(1R, 2S)

The platinum(IV) complexes of the formulae (1) to (3) according to this invention, therefore, include some different stereo-isomers as shown above, depending on the configuration 1,2-cyclohexanediamine or 2-(aminomethyl)cyclohexylamine which forms a ligand to the platinum atom.

According to particular embodiments of this invention, there are provided five types of the platinum(IV) complexes as described below :

(1) A complex of the formula (2) in which the dach moiety is a ligand selected from cis-1,2-cyclohexanediamine, trans-d-1,2-cyclohexanediamine and trans-ℓ-1,2-cyclohexadiamine,

(2) A complex of the formula (3) in which the dach moiety is a ligand selected from cis-1,2-cyclohexanediamine, trans-d-1,2-cyclohexanediamine and trans-ℓ-1,2-cyclohexanediamine

(3) A complex of the formula (1) in which the amcha moiety is a ligand selected from cis-dℓ-2-(aminomethyl)cyclohexylamine and trans-dℓ-2-(aminomethyl)cyclohexylamine and B, B′ and D are each a chloro group.

(4) A complex of the formula (1) in which the amcha moiety is a ligand selected from cis-dℓ-2-(aminomethyl)cyclohexylamine, and B and B′ are each a chloro group, and D is hydroxo group (OH) or a nitrato group ($NO_3$).

(5) A complex of the formula (1) in which the amcha moiety is a ligand selected from cis-dℓ-2-(aminomethyl)cyclohexylamine and trans-dℓ-2-(aminomethyl)cyclohexylamine and B, B′ taken together form a group of the formula

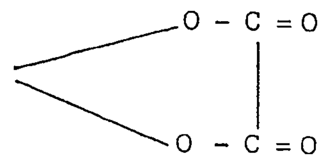

and D is a hydroxo group (OH), a chloro group (Cℓ) or a nitrato group ($NO_3$).

With reference to the attached drawings :

Fig 1 is infrared absorption spectrum (KBr) of trans-(Cℓ)-oxalato-dichloro-(trans-ℓ-dach)platinum(IV) according to this invention.

Fig 2 is infrared absorption spectrum (KBr) of trans-(Cℓ)-malonato-dichloro(trans-ℓ-dach)platinum(IV) according to this invention.

Fig 3 is infrared absorption spectrum (KBr) of tetrachloro-(cis-dℓ-amcha)platinum(IV) according to this invention.

Fig 4 is infrared absorption spectrum (KBr) of tetrachloro-(trans-dℓ-amcha)platinum(IV) according to this invention.

Fig 5 is infrared absorption spectrum (KBr) of trans-(OH)-dichloro-dihydroxo(cis-dℓ-amcha)platinum(IV) according to this invention.

Fig 6 is infrared absorption spectrum (KBr) of trans-(OH)-dichloro-dihydroxo(trans-dℓ-amcha)platinum(IV) according to this invention.

Fig 7 is infrared absorption spectrum (KBr) of trans-(OH)-oxalato-dihydroxo(cis-dℓ-amcha)platinum(IV) according to this invention.

Fig 8 is infrared absorption spectrum (KBr) of trans-($NO_3$)-oxalato-dinitrato(cis-dℓ-amcha)platinum(IV) according to this invention.

Fig 9 is infrared absorption spectrum (KBr) of trans-($NO_3$)-oxalato-dinitro(trans-dℓ-amcha)platinum(IV) according to this invention.

Fig 10 is infrared absorption spectrum (KBr) of trans-(Cℓ)-oxalato-dichloro(cis-dℓ-amcha)platinum(IV) according to this invention.

The platinum(IV) complex of the general formulae (1) to (3) according to this invention may be prepared by one of the following two processes, (A) and (B).

Process (A) ;

In this process, the platinum(II) complex of the general formula (II)

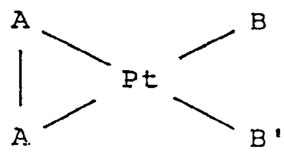

(II)

wherein the moiety

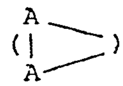

is a dach or amcha ligand, B and B'are as in formula (1), is reacted with chlorine gas or hydrogen peroxide. The starting material, namely the compound of the general formula (II) may be prepared according to a known method, for example, the method disclosed in documents JP-B-41077/85, US-C-4,169,846 and US-C-4,255,347.

Process (B) ;

In this process, such a starting compound of the general formulae (1) to (3) where platinum is substituted by two hydroxo groups, which has been obtained in the process (A) above, is reacted with a diluted hydrochloric acid or diluted nitric acid, so that the hydroxo groups present in the starting compound is converted into a chloro group (Cℓ) or nitrato group ($NO_3$), to give such compounds of the formulae (1) to (3) with Cℓ or $NO_3$ substituents, according to the invention.

The platinum(IV) complexes according to this invention thus obtained can be identified by their infrared absorption spectra as shown in Figures 1 to 10 of the attached drawings. The method for the identification of the compounds is explained below.

All the platinum(IV) complexes according to this invention commonly exhibit IR absorption peaks due to the carrier ligand present in the complexes, namely, IR absorption peaks at 3200 to 3100 $cm^{-1}$ due to $\nu NH_2$ at 2900 $cm^{-1}$ due to νCH and at 1600 $cm^{-1}$ or thereabout due to $\nu NH_2$.

By checking the shift of the characteristic infrared absorption peak in a particular platinum(IV) complex, this platinum(IV) complex can be identified about what is the configuration of the leaving groups relative to the platinum(IV) atoms as illustrated below.

Trans-(OH)-dichloro-dihydroxo(amcha)platinum(IV) exhibit an IR absorption peak at 3500 $cm^{-1}$ due to νOH, as shown in Fig. 5 of the attached drawings.

Trans-(OH)-oxalato-dihydroxo(amcha)platinum(IV) exhibit an IR absorption peak at 3500 $cm^{-1}$ due to νOH, and exhibit strong absorption peaks at 1700 $cm^{-1}$ and 1400 $cm^{-1}$, due to νC=0 and νC-0, as shown in Fig. 7.

Trans-($NO_3$)-oxalato-dinitrato(amcha)platinum(IV), as shown in Fig. 9 of the attached drawings, exhibit IR absorption peaks at 1600 $cm^{-1}$ and 1400 $cm^{-1}$ due to νC=O and νC-O, and IR absorption peaks at 1550 $cm^{-1}$, 1280 $cm^{-1}$ and 950 $cm^{-1}$ due to $\nu NO_3$.

Trans-(Cℓ)-oxalato-dichloro(amcha)platinum(IV) exhibit IR absorption peaks at 1700 $cm^{-1}$ and 1380 $cm^{-1}$ due to νC=O and νC-O, as shown in Fig.10 of the attached drawings.

The new platinum(IV) complexes according to this invention exhibit anti-tumor activity against experimental tumors on mouse, such as L1210, P388 and S180A (ascites-tumor), and therefore are useful in chemotherapeutics of tumors. The new platinum(IV) complexes of this invention can be administered orally, intramuscularly or intravenously. They can be formulated into capsules, powders, pellets or injections.

Suitable dosage of the platinum(IV) complexes of this invention is about 1 to 400 mg/kg/day.

The production of the novel platinum(IV) complexes of the formula (I) according to this invention is now illustrated with reference to the following examples. The data of elementary analysis and yield of the organo-platinum(IV) complex as produced in the Examples are listed in Table 1 hereinafter. Compound Numbers given in Table 1 are corresponding to the Example Nos. in which the compound indicated was prepared.

Example 1

Preparation of trans-(Cℓ)-oxalato-dichloro(cis-dach)platinum(IV).

Oxalato(cis-dach)platinum(II) (0,5 g) was suspended in water (5 ml), into which was then passed chlorine gaz for 45 minutes under heating at 70°C on a water bath. The resulting suspension was passed through with air stream for 10 minutes to remove the residual chlorine gas therefrom, so that a transparent, yellow colored

reaction solution was obtained. The reaction solution was ice-cooled to deposit a precipitate which was then recovered by filtration. The solid product obtained was dried under reduced pressure, to afford the titled complex.

Example 2

Preparation of trans-(Cℓ)-oxalato-dichloro(trans-d-dach)platinum(IV). Oxalato(trans-d-dach)platinum(II) as a starting materiel was reacted with chlorine gas and then processed in the same manner as in Example 1 above, to afford the titled complex.

Example 3

Preparation of trans-(Cℓ)-oxalato-dichloro(trans-ℓ-dach)-platinum(IV).

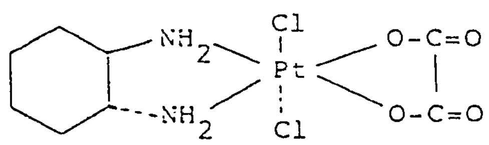

Oxalato(trans-ℓ-dach)platinum(II) as a starting materiel was reacted with chlorine gas and then processed in the same manner as in Example 1, to afford the titled complex.

Example 4

Preparation of trans-(Cℓ)-malonato-dichloro(cis-dach)platinum(IV).

Trans-(OH)-malonato-dihydroxo(cis-dach)platinum(IV) (0,54 g) was suspended in water (20 ml), to which was then added 0.100 N aqueous hydrochloric acid (24 ml). The resulting suspension was stirred at room temperature for 30 minutes and allowed to stand until a transparent reaction solution was formed. The reaction solution was concentrated and the residue was dried under reduced pressure. The dried residue was dissolved in ethanol (30 ml) at 50°C and the solution was filtrated to remove a small amount of colorless, insoluble matter. The filtrate was concentrated and dried under reduced pressure, and a yellow solid residue obtained was washed with a small volume of ethanol and dried, to afford the titled complex.

Example 5

Preparation of trans-(Cℓ)-malonato-dichloro(trans-d-dach)platinum(IV).

Trans-(OH)-malonato-dihydroxo(trans-d-dach)platinum(IV) as a starting materiel was reacted with hydrochloric acid and then processed in the same manner as in Example 4 above, to afford the titled complex.

Example 6

Preparation of trans-(Cℓ)-malonato-dichloro(trans-ℓ-dach)-platinum(IV).

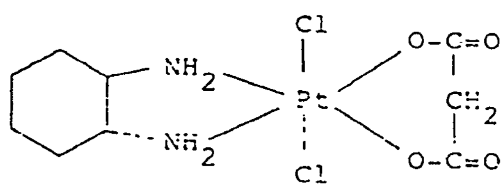

Trans-(OH)-malonato-dihydroxo(trans-ℓ-dach)platinum(IV) as a starting materiel was reacted with aqueous hydrochloric acid and then processed in the same manner as in Example 4, to afford the titled complex.

Example 7

Preparation of tetrachloro(cis-ℓ-amcha)platinum(IV).

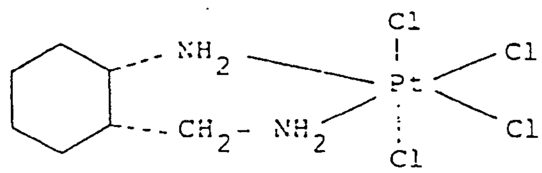

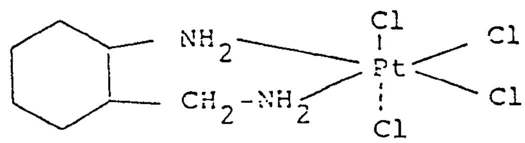

Dichloro(cis-dℓ-amcha)platinum(II) (0,5 g) (see U.S. patent No. 4,255,347) was suspended in water (5 ml), into which was then passed chlorine gas for 45 minutes under heating at 70°C on a water bath. The resulting suspension was passed through with air stream for 10 minutes to remove the remaining chlorine gas therefrom. The resultant yellow and transparent reaction solution was ice-cooled to deposit a precipitate, and this precipitate was recovered by filtration and dried under reduced pressure, to afford the titled complex.

Example 8

Preparation of tetrachloro(trans-dℓ-amcha)platinum(IV).

Dichloro(trans-dℓ-amcha)platinum(IV) as as starting materiel was reacted with chlorine gas and then processed in the same manner as in Example 7 above, to afford the titled complex.

Example 9

Preparation of trans-(OH)-dichloro-dihydroxo(cis-dℓ-amcha)platinum(IV).

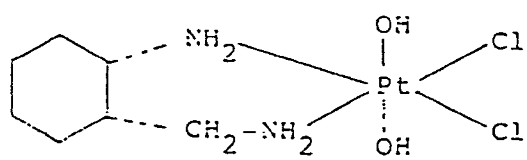

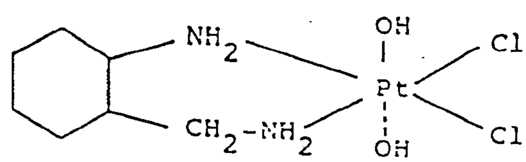

Dichloro(cis-dℓ-amcha)platinum(II) (0,5 g) was suspended in water (5 ml), to which was then added 30% aqueous hydrogen peroxide (25 ml) under heating at 70°C on a water bath. The resulting suspension was stirred for 45 minutes and allowed to stand. The resultant transparent and yellow reaction solution was concentrated to 3 ml under reduced pressure. The concentration was admixed with ethanol to deposit a precipitate which was recovered by filtration. The titled complex was thus obtained.

Example 10

Preparation of trans-(OH)-dichloro-dihydroxo(trans-dℓ-amcha)platinum(IV) Dichloro(trans-dℓ-amcha)platinum(II) as a starting materiel was reacted with aqueous hydrogen peroxide and then processed in the same manner as in Example 9 above, to afford the titled complex.

Example 11

Preparation of trans-($NO_3$)-dichloro-dinitrato(cis-dℓ-amcha)platinum(IV)

Trans-(OH)-dichloro-dihydroxo(cis-dℓ-amcha)platinum(IV) (0,5 g) was suspended in water (5 ml), to which

was then added 0.1 N aqueous nitric acid (25 ml). The resulting suspension was stirred at room temperature for 1 hour to effect the reaction. The resultant transparent reaction solution was concentrated under reduced pressure. The solid residue was washed with a small volume of ethanol and dried under reduced pressure, to obtain the titled complex.

Example 12

Preparation of trans-($NO_3$)-dichloro-dinitrato(trans-dℓ-amcha)platinum(IV)

Trans-(OH)-dichloro-dihydroxo(trans-dℓ-amcha)platinum(IV) as a starting material was reacted with aqueous nitric acid and then processed in the same manner as in Example 11 above, to afford the titled complex.

Example 13

Preparation of trans-(OH)-oxalato-dihydroxo(cis-dℓ-amcha)platinum(IV)

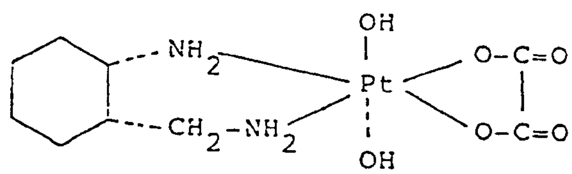

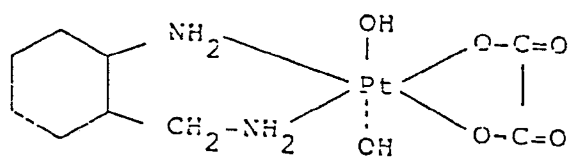

Oxalato(cis-dℓ-amcha)platinum(II) (1.0 g) (see U.S. patent No. 4,255,347) was suspendend in water (10 ml), to which was then added 30% aqueous hydrogen peroxide (50 ml) under heating at 70°C on a water bath, and the resulting suspension was further stirred for 45 minutes to effect the reaction. The resultant clear reaction solution was concentrated to 5 ml under reduced pressure. The concentrate was admixed with ethanol to deposit a colorless precipitate. This precipitate was recovered by filtration and dried under reduced pressure, to afford the titled complex.

Example 14

Preparation of trans-(OH)-oxalato-dihydroxo(trans-dℓ-amcha)platinum(IV)

Oxalato(trans-dℓ-amcha)platinum(II) as a starting material was reacted with aqueous hydrogen peroxide and then processed in the same manner as in Example 13 above, to afford the titled complex.

Example 15

Preparation of trans-($NO_3$)-oxalato-dinitrato(cis-dℓ-amcha)platinum(IV)

Trans-(OH)-oxalato-dihydroxo(cis -dℓ-amcha)platinum(IV) (1.0 g) was suspended in water (10 mg), to which was then added 0.1 N aqueous nitric acid (50 ml), and the resulting suspension was further stirred at room temperature for 1 hour to effect the reaction. The resultant reaction solution was concentrated under reduced pressure. The concentrate was washed with a small volume of ethanol and dried under reduced pressure, to afford the titled complex.

Example 16

Preparation of trans-($NO_3$)-oxalato-nitrato(trans-dℓ-amcha)platinum(IV)

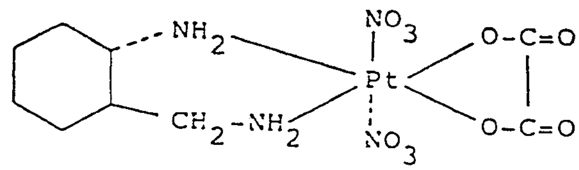

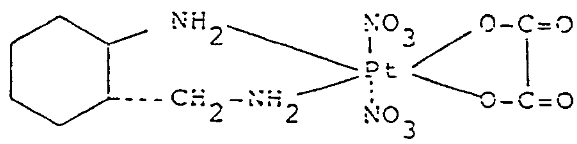

Trans-(OH)-oxalato-dihydroxo(trans-dℓ-amcha)platinum(IV) as a starting material was reacted with aqueous nitric acid and then processed the same manner as in Example 15 above, to afford the titled complex.

Example 17

Preparation of trans-(Cℓ)-oxalato-dichloro(cis-dℓ -amcha)platinum(IV) Oxalato(cis-dℓ-amcha)platinum(II) (1.0 g) was suspended in water (10 ml), into which was then passed chlorine gas for 1 hour under heating at 70°C on a water bath. The resulting suspension was passed through with air stream for 10 minutes to remove the remaining chlorine gas therefrom. The resultant clear and yellow reaction solution was ice-cooled to deposit a precipitate, and the precipitate was recovered by filtration and dried under reduced pressure, to afford the titled complex.

Example 18

Preparation of trans-(Cℓ)-oxalato-dichloro(cis-dℓ-amcha)platinum(IV)

Oxalato(trans-dℓ-amcha)platinum(IV) as a starting material was reacted with chlorine gas and then processed in a manner similar to Example 17, to afford the titled complex.

The elementary analysis value and the yield of the respective platinum(IV) complex according to this invention as obtained in the Examples 1 to 18 above, are summarized in Table 1 below.

Table 1

| Compound No. (Example No.) | Found value (%) | | | Calculated value (%) | | | Yield (%) |
|---|---|---|---|---|---|---|---|
| | H | C | N | H | C | N | |
| 1 | 2.87 | 20.37 | 6.24 | | | | 70 |
| 2 | 3.14 | 20.43 | 6.23 | 3.00 | 20.56 | 6.13 | 68 |
| 3 | 3.12 | 20.45 | 6.02 | | | | 72 |
| 4 | 3.29 | 22.35 | 5.73 | 3.32 | 22.41 | 5.81 | 70 |
| 5 | 3.42 | 22.52 | 5.92 | | | | 75 |
| 6 (1/2-hydrate) | 3.85 | 21.99 | 5.44 | 3.88 | 21.92 | 5.63 | 80 |
| 7 | 3.22 | 18.42 | 5.99 | 3.45 | 18.14 | 6.05 | 70 |
| 8 | 3.42 | 18.33 | 5.99 | | | | 65 |
| 9 | 4.59 | 19.93 | 6.40 | 4.22 | 19.67 | 6.56 | 52 |
| 10 | 3.96 | 19.63 | 6.39 | | | | 50 |
| 11 | 3.21 | 16.05 | 10.63 | 3.09 | 16.22 | 10.81 | 75 |
| 12 | 3.15 | 16.32 | 10.92 | | | | 72 |
| 12 (mono-hydrate) | 4.22 | 22.95 | 6.11 | 4.32 | 23.32 | 6.05 | 63 |
| 14 | 4.03 | 24.10 | 6.27 | 4.04 | 24.27 | 6.29 | 47 |
| 15 | 3.48 | 18.73 | 10.27 | 2.99 | 20.19 | 10.47 | 40 |
| 16 (di-hydrate) | 3.36 | 18.67 | 10.12 | 3.50 | 18.91 | 9.81 | 35 |
| 17 (tri-hydrate) | 3.42 | 21.21 | 5.33 | 3.61 | 21.64 | 5.61 | 63 |
| 18 | 3.66 | 22.16 | 5.57 | 3.33 | 22.45 | 5.82 | 81 |

Experiment 1

Antitumor activity of the platinum(IV) complex of this invention on mice against Leukemia L-1210 is now estimated.

To test the anti-tumor activity of the platinum(IV) complexes according to this invention, $10^5$ cells/mouse of Leukemia L-1210 were transplanted by intraperitoneal injection to groups (6 mice in each group) of CDFI mice. On the same day of the transplantation, and 5 days and 9 days after the transplantation of Leukemia L-1210 cells, the platinum(IV) complex under test was administered by intraperitoneal injection to the test mice. The anti-tumor activity of the test platinum(IV) complex was evaluated by means of rate (%) of prolongation of mean survival days of the treated mice, in term of the values of T/C %, i.e., the value of 100 times the mean survival period of the groups of mice treated with the test platinum complex, divided by the mean survival period of the comparative groups of mice which were not treated with the test platinum complex. The test results are as shown in Table 2 below. In Table 2, the T/C (%) values of higher than 125% (the numerical value as underlined) means that the tested platinum complex has a substantial anti-tumor activity.

In Table 2, the numerical figure given in parenthesis denotes the number of the mice as entirely cured in each group of mice treated. The term "T" denotes that the incurred decrease in body weight of the treated mice exceeded the lowest limit value (-4 g) for judgement of the toxic effects of the test compound. The test results shown in Table 2 demonstrates that the platinum(IV) complexes of this invention have a significant antitumor activity.

Table 2

| Compound No. of platinum (IV) complex | Rate (%) of prolongation of mean survival period (T/C, %) | | | | | | | | |
|---|---|---|---|---|---|---|---|---|---|
| | Dose (mg/kg) | | | | | | | | |
| | 400 | 200 | 100 | 50 | 25 | 12.5 | 6.25 | 3.12 | 1.56 |
| 3 | | | | 153 | 352(3) | 311(3) | | | |
| 6 | | | 0 | 243(2) | 286(2) | | | | |
| 7 | | | | | | 224(1) | 153 | 192(1) | |
| 8 | | | | "T"256(1) | 300(3) | 217(1) | 170 | 124 | 111 |
| 9 | | | | 211 | 212 | 132 | | | |
| 10 | | | | 147 | 138 | 119 | | | |
| 11 | | | | "T" 75 | "T"87 | "T"143 | | | |
| 12 | 245(1) | 221(1) | 181 | 117 | 100 | 102 | | | |
| 14 | 250(1) | 164 | 129 | 117 | 105 | 115 | | | |
| 15 | | | 0 | 75 | 81 | | | | |
| 16 | | | 268(1) | 253(1) | 143 | | | | |
| 17 | | | | 95 | 95 | 176 | | | |
| 18 | | | | 119 | 309(3) | 306(2) | | | |

## Claims

1. An antitumor platinum(IV) complex, characterized in that it has a general formula

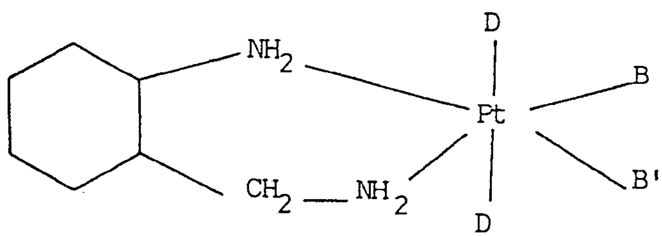

wherein B and B' taken together denote a group of the formula

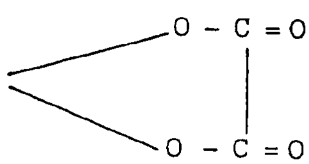

or a group of the formula

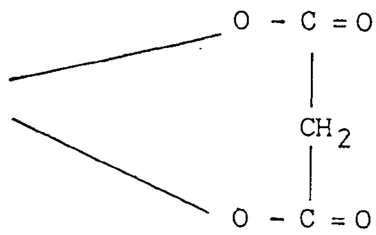

or B and B′ each denote a chloro group, and D denotes a chloro group, a nitrato group or a hydroxy group

2. An antitumor platinum(IV) complex, characterized in that it has a general formula

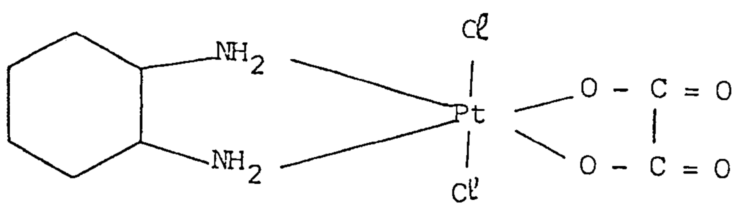

3. An antitumor platinum(IV) complex having a general formula

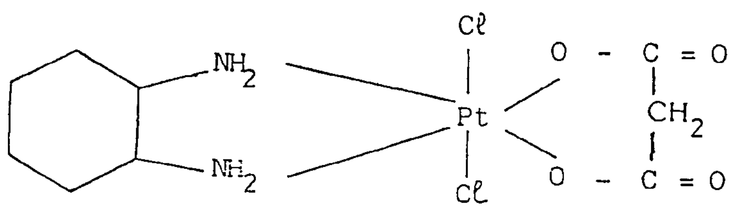

4. A compound as claimed in claim 1, characterized in that it is trans-(Cℓ)-oxalato-dichloro(trans-dℓ-1-amino-2-aminomethylcyclohexane) platinum(IV) according to the following formula

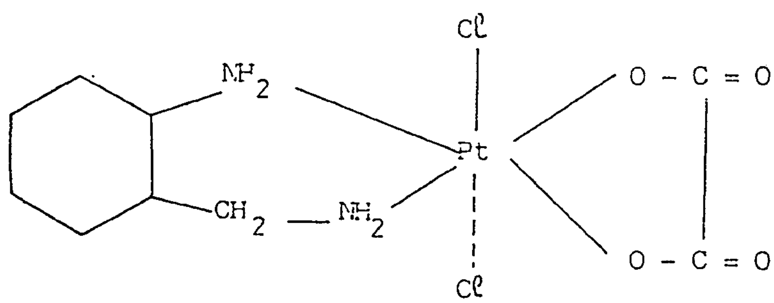

5. A compound as claimed in claim 2 characterized in that it is trans-(Cℓ)-oxalato-dichloro(trans-ℓ-1,2-diaminocyclohexane)platinum(IV) of the formula

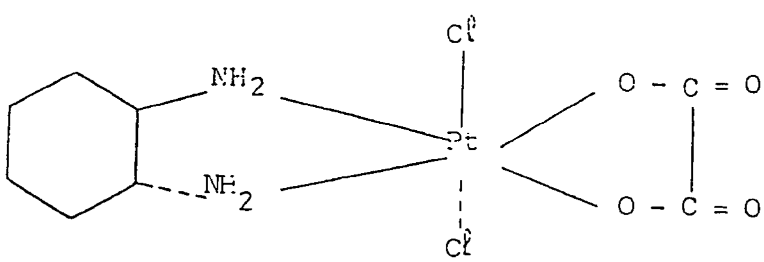

6. A compound as claimed in claim 3 characterized in that it is trans-(Cℓ)-malonato-dichloro(trans-ℓ-1,2-diaminocyclohexane)platinum(IV) of the formula

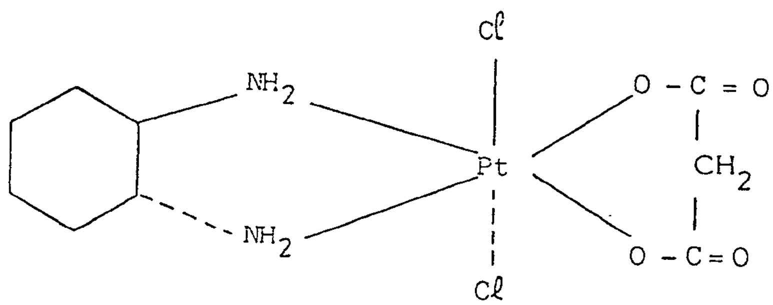

## Ansprüche

1. Anti-Tumor Platin(IV)-Komplex, gekennzeichnet durch die allgemeine Formel

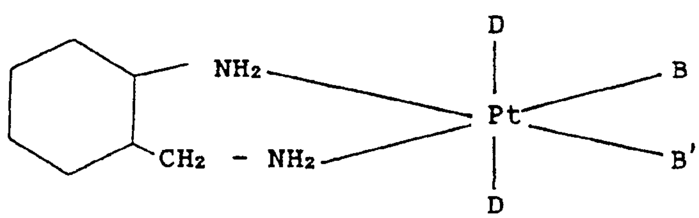

wobei B und B′, zusammengenommen, eine Gruppe der Formel

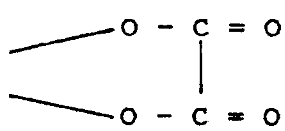

oder eine Gruppe der Formel

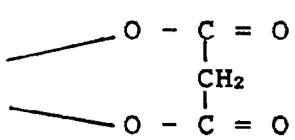

oder B und B′ jeweils eine Chloro Gruppe bezeichnen, und D eine Chloro-, Nitrato- oder eine Hydroxylgruppe bezeichnet.

2. Anti-Tumor Platin(IV)-Komplex, gekennzeichnet durch die allgemeine Formel

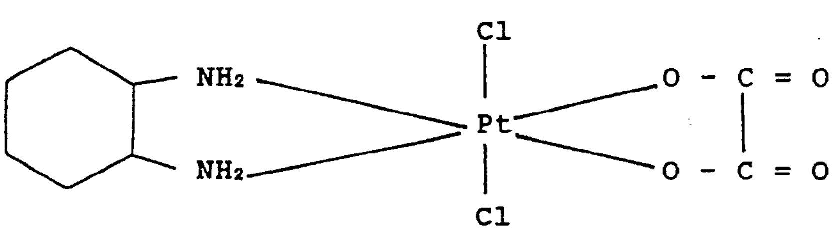

3. Anti-Tumor Platin(IV)-Komplex mit der allgemeinen Formel

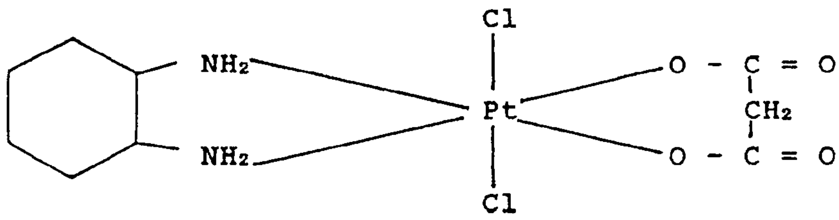

4. Verbindung nach Anspruch 1, dadurch gekennzeichnet, daß sie trans-(Cl)-Oxalato-dichloro(trans-dl-1-amino-2-aminomethylcyclohexan)platin(IV) mit folgender Formel

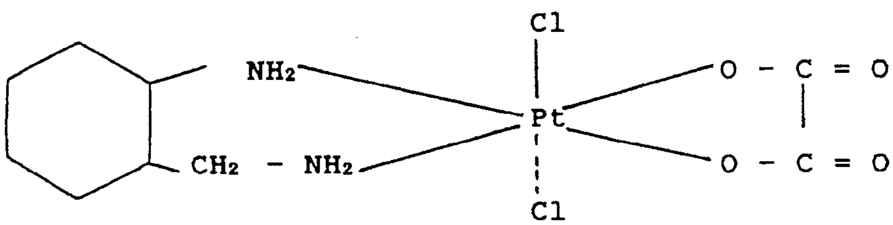

ist.

5. Verbindung nach Anspruch 2, dadurch gekennzeichnet, daß sie trans-(Cl)-Oxalato-dichloro(trans-l-1,2-diaminocyclohexan)platin(IV) mit der Formel

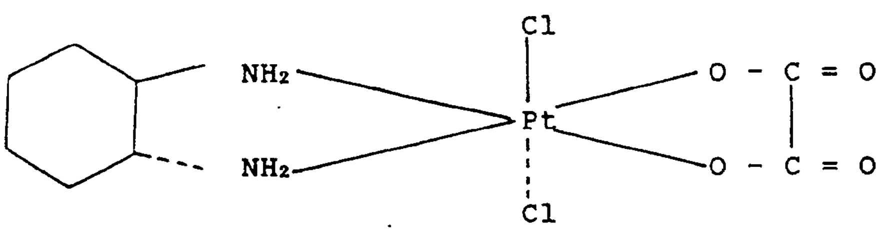

ist.

6. Verbindung nach Anspruch 3, dadurch gekennzeichnet, daß sie trans-(Cl)-Malonato-dichloro(trans-l-1,2-diaminocyclohexan)platin(IV) mit der Formel

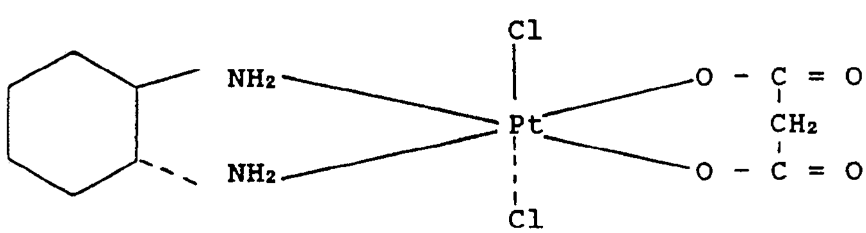

ist.

## Revendications

1. Complexe au platine (IV) antitumoral, caractérisé en ce qu'il a la formule générale :

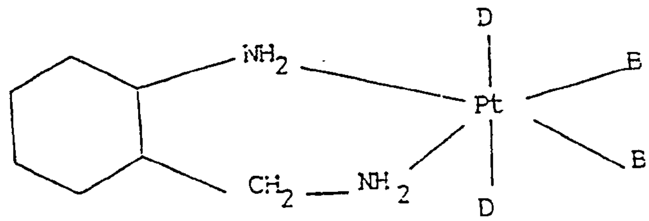

dans laquelle B et B′ pris ensemble, représentent un groupement de formule

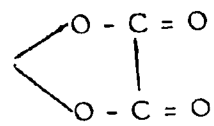

ou un groupement de formule

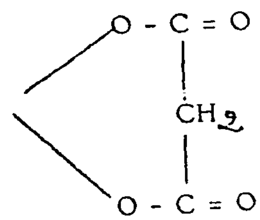

ou B et B′ représentent chacun un groupement chloro, et D represente un groupement chloro, un groupement nitro ou un groupement hydroxyle.

2. Complexe au platine (IV) antitumoral, caractérisé en ce qu'il a la formule générale

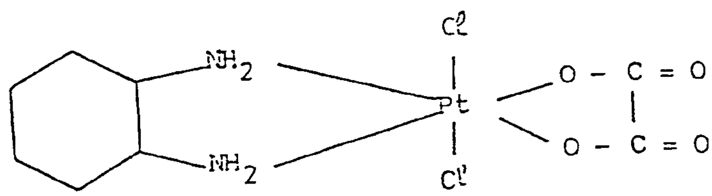

3. Complexe au platine (IV) antitumoral, ayant la formule générale

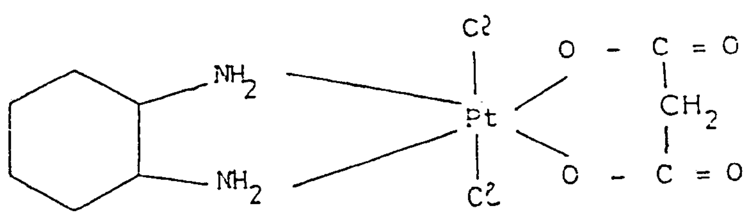

4. Composé selon la revendication 1, caractérisé en ce que c'est le platine (IV)-trans-(Cℓ)-oxalato-dichloro(trans-dℓ-1-amino-2-aminomethylcyclohexane) selon la formule suivante

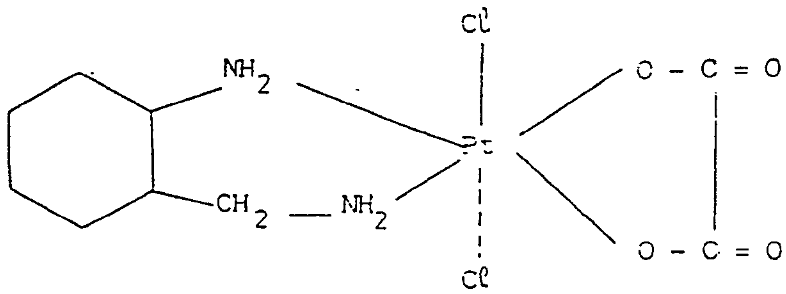

5. Composé selon la revendication 2, caractérisé en ce que c'est le platine (IV)-trans(Cℓ)-oxalato-dichloro(trans-ℓ-1,2-diaminocyclohexane) de formule

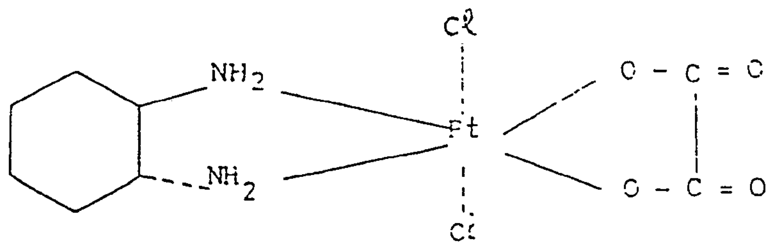

6. Composé selon la revendication 3, caractérisé en ce que c'est le platine (IV)-trans(Cℓ)malonato-dichloro(trans-ℓ-1,2-diaminocyclohexane) de formule

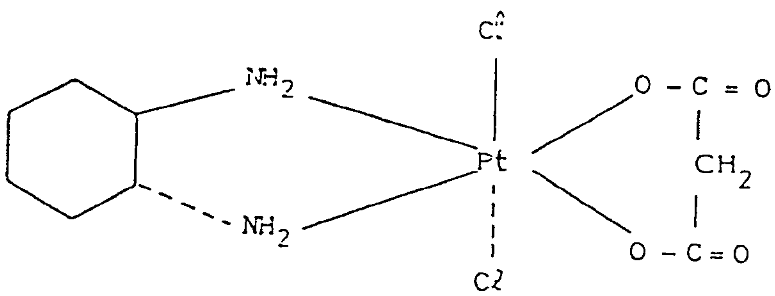

FIG. 1

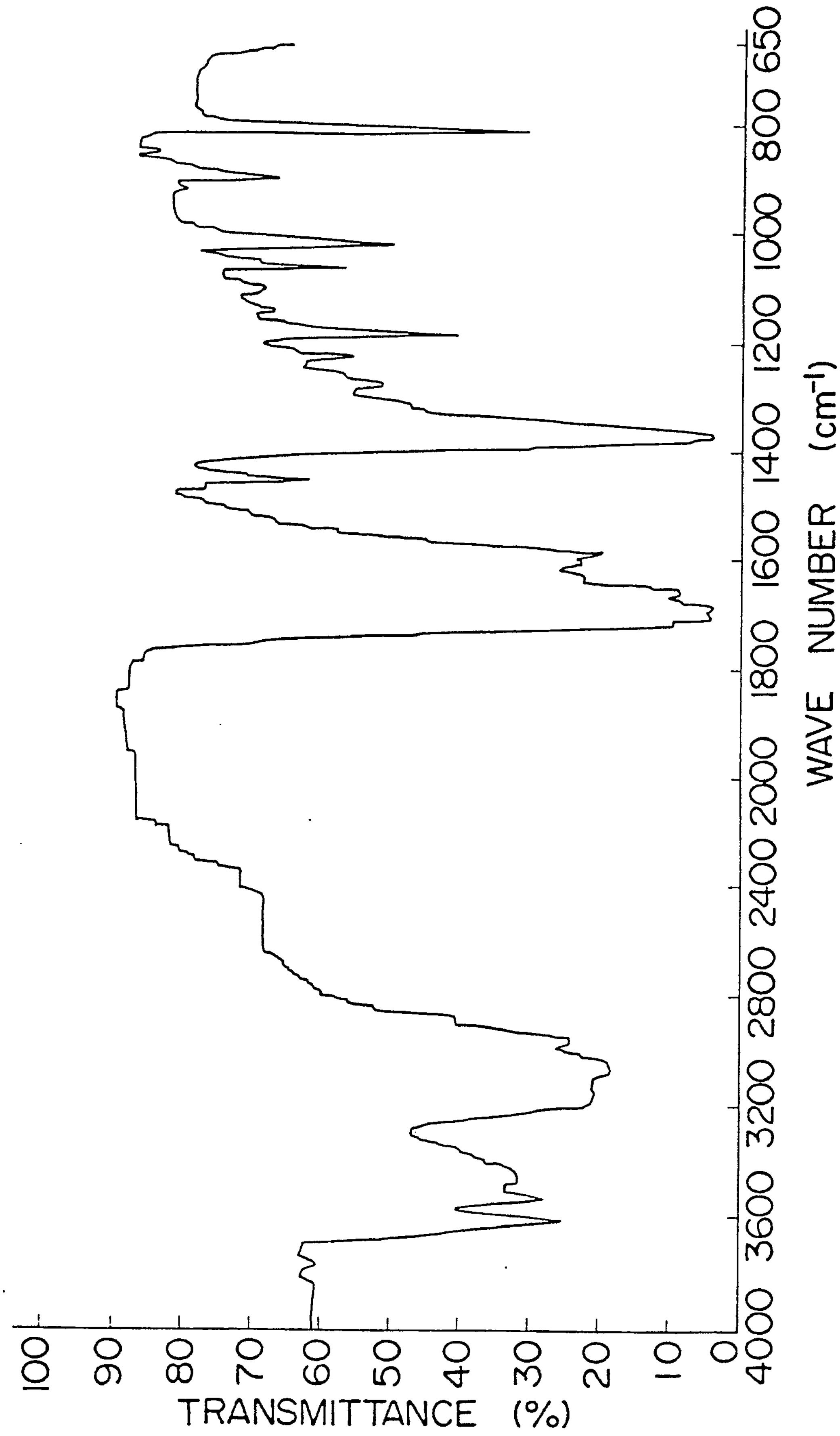

TRANSMITTANCE (%)
100
90
80
70
60
50
40
30
20
10
0
WAVE NUMBER (cm⁻¹)
4000
3600
3200
2800
2400
2000
1800
1600
1400
1200
1000
800
650

FIG. 2

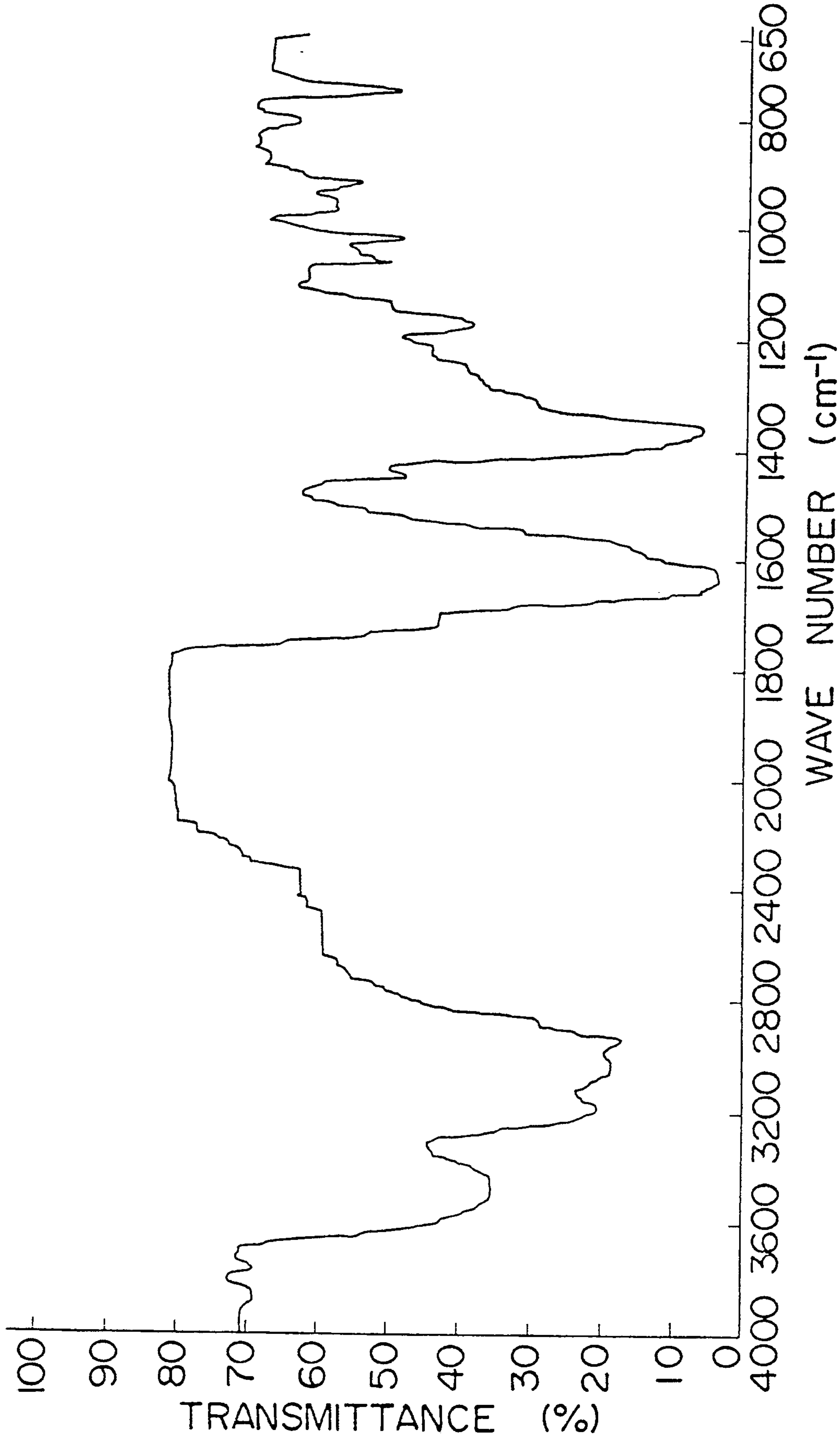
TRANSMITTANCE (%)
0
10
20
30
40
50
60
70
80
90
100
WAVE NUMBER (cm⁻¹)
4000
3600
3200
2800
2400
2000
1800
1600
1400
1200
1000
800
650

FIG. 3

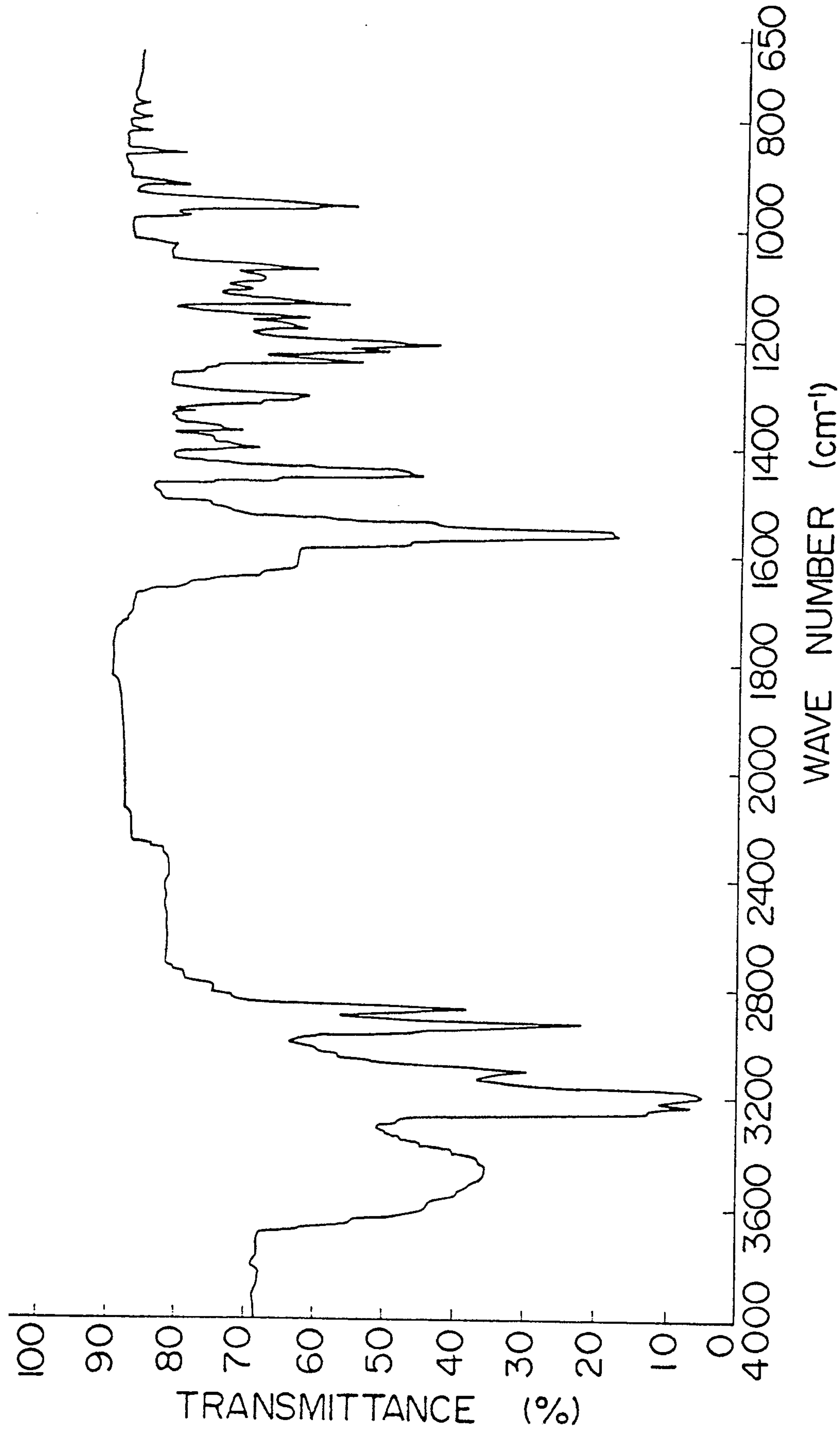
100
90
80
70
60
50
40
30
20
10
0
TRANSMITTANCE (%)
4000
3600
3200
2800
2400
2000
1800
1600
1400
1200
1000
800
650
WAVE NUMBER (cm⁻¹)

FIG. 4

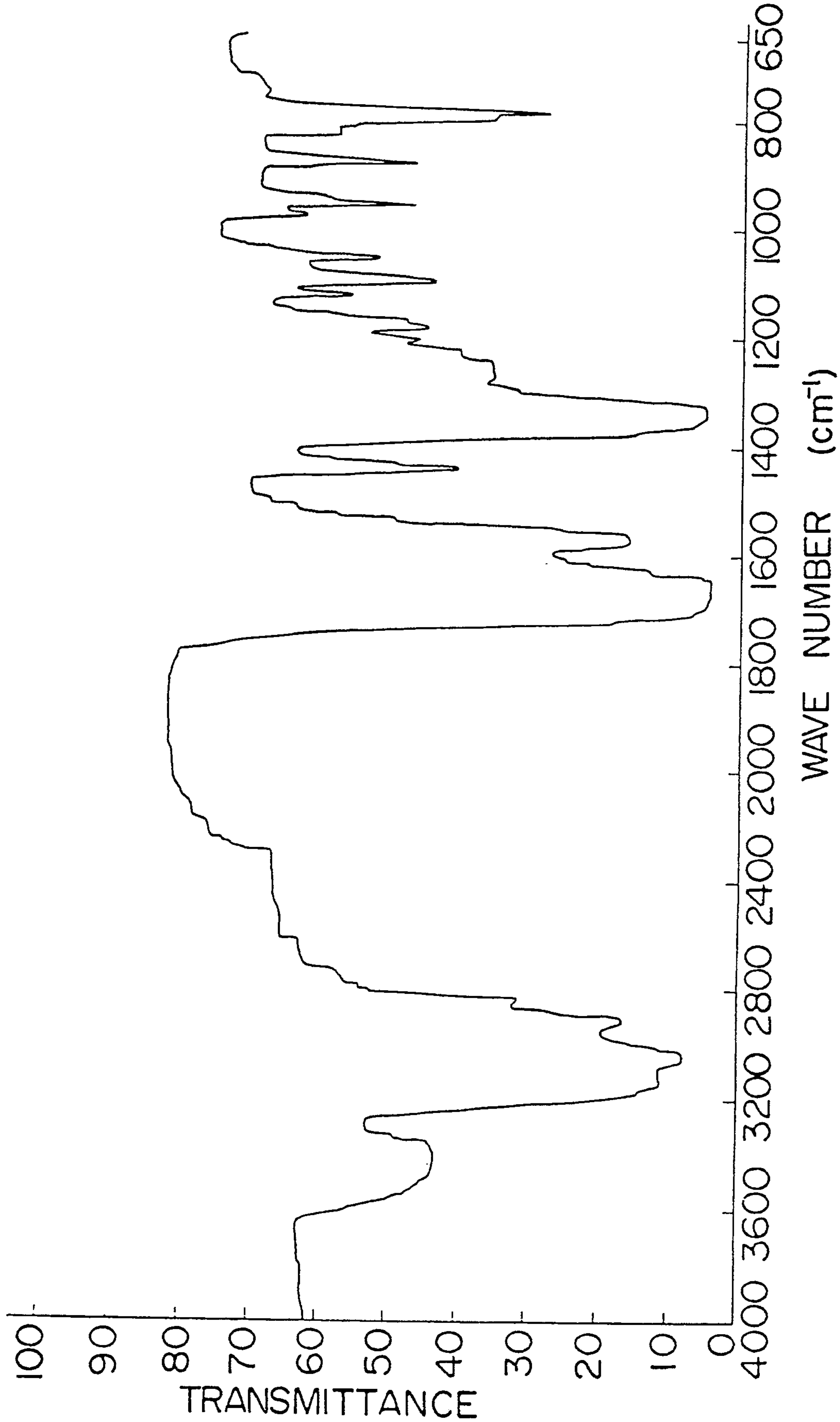
TRANSMITTANCE
100
90
80
70
60
50
40
30
20
10
0
WAVE NUMBER (cm$^{-1}$)
4000
3600
3200
2800
2400
2000
1800
1600
1400
1200
1000
800
650

FIG. 5

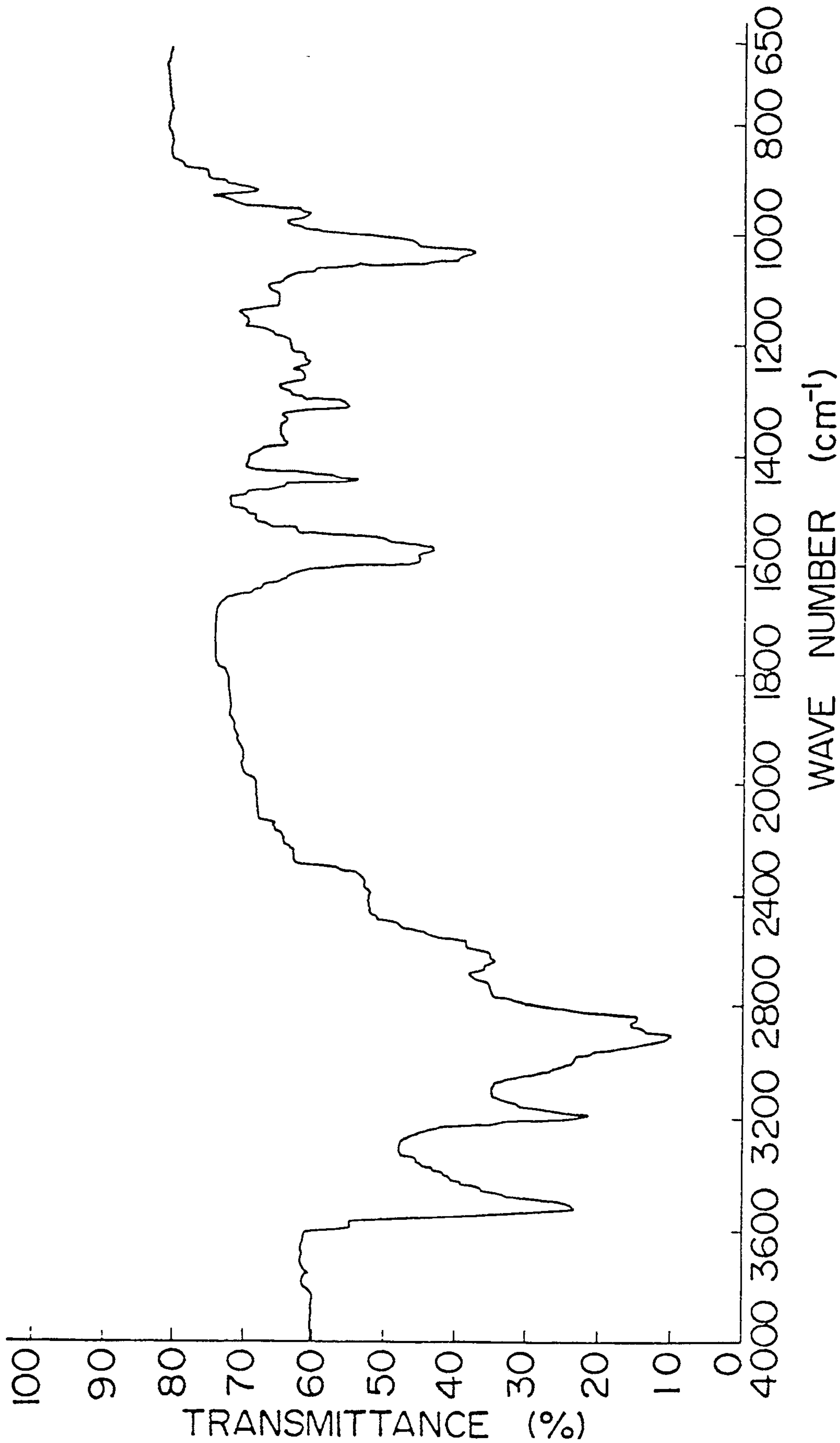
TRANSMITTANCE (%)
100
90
80
70
60
50
40
30
20
10
0
4000
3600
3200
2800
2400
2000
1800
1600
1400
1200
1000
800
650
WAVE NUMBER (cm$^{-1}$)

FIG. 6

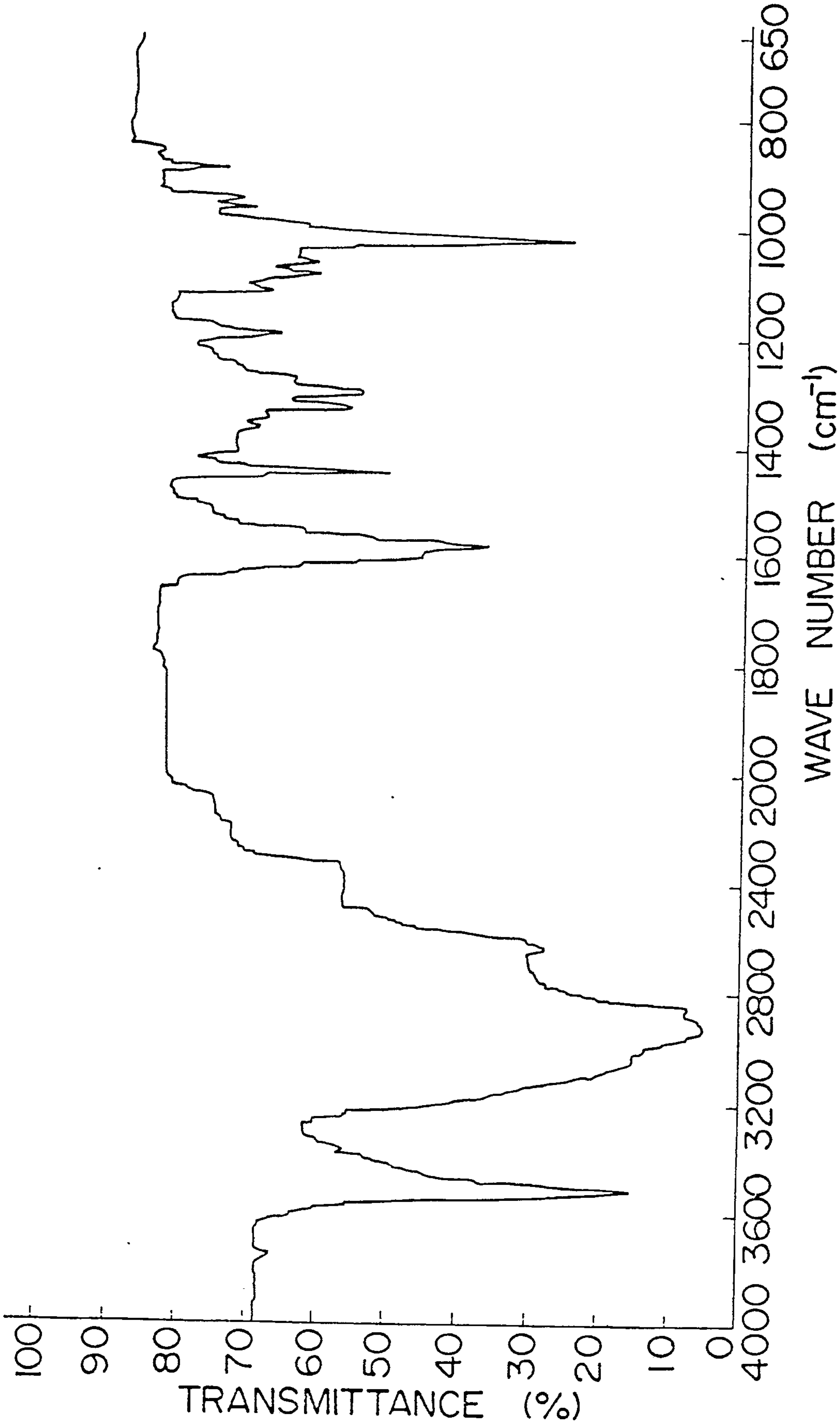
TRANSMITTANCE (%)
100 90 80 70 60 50 40 30 20 10 0
WAVE NUMBER (cm⁻¹)
4000 3600 3200 2800 2400 2000 1800 1600 1400 1200 1000 800 650

FIG. 7

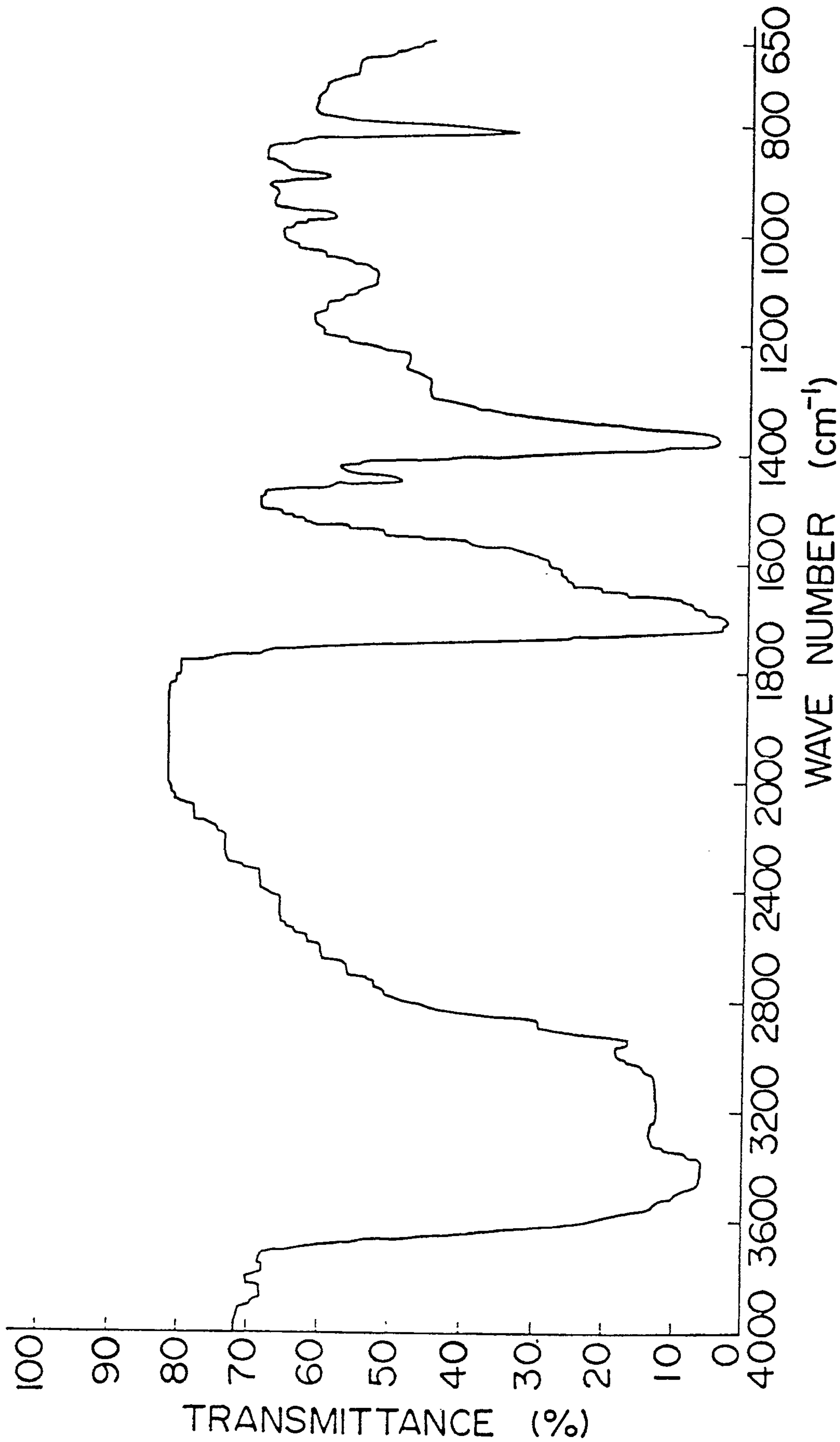
TRANSMITTANCE (%)
0
10
20
30
40
50
60
70
80
90
100
WAVE NUMBER (cm⁻¹)
4000
3600
3200
2800
2400
2000
1800
1600
1400
1200
1000
800
650

# FIG. 8

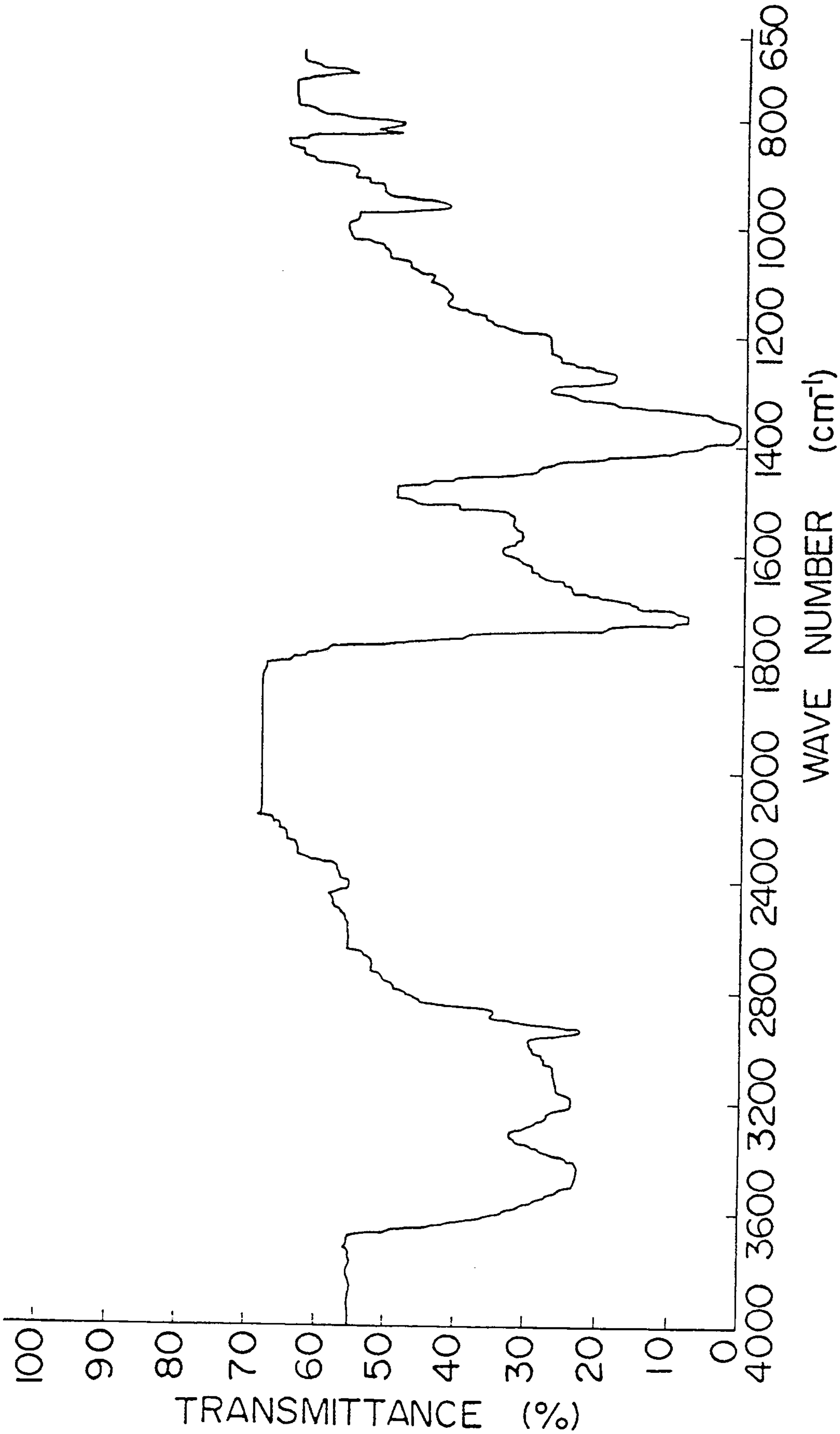
TRANSMITTANCE (%)
0 10 20 30 40 50 60 70 80 90 100
WAVE NUMBER (cm⁻¹)
4000 3600 3200 2800 2400 2000 1800 1600 1400 1200 1000 800 650

FIG. 9

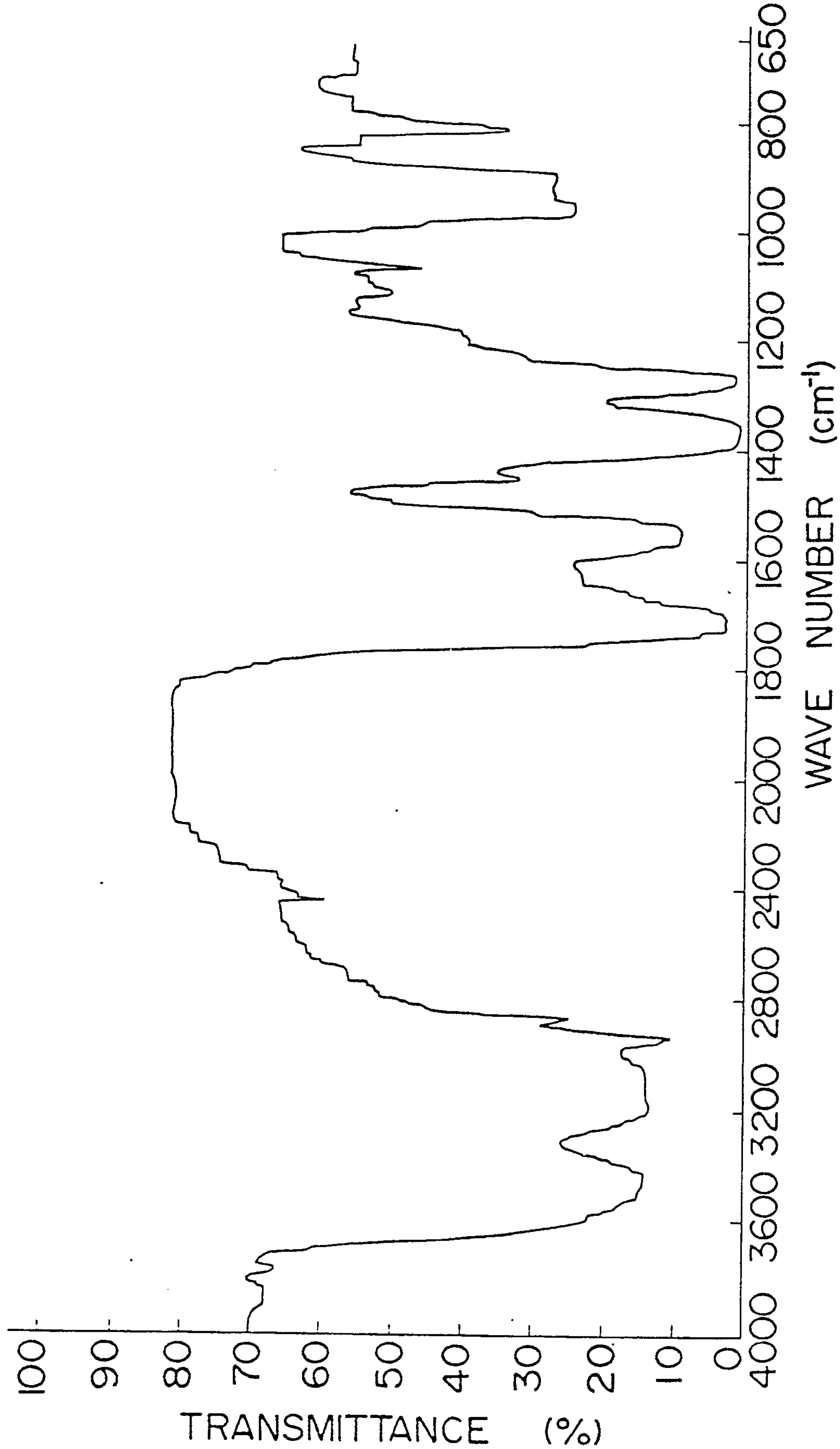
TRANSMITTANCE (%)
0
10
20
30
40
50
60
70
80
90
100
WAVE NUMBER $(cm^{-1})$
4000
3600
3200
2800
2400
2000
1800
1600
1400
1200
1000
800
650

FIG.10

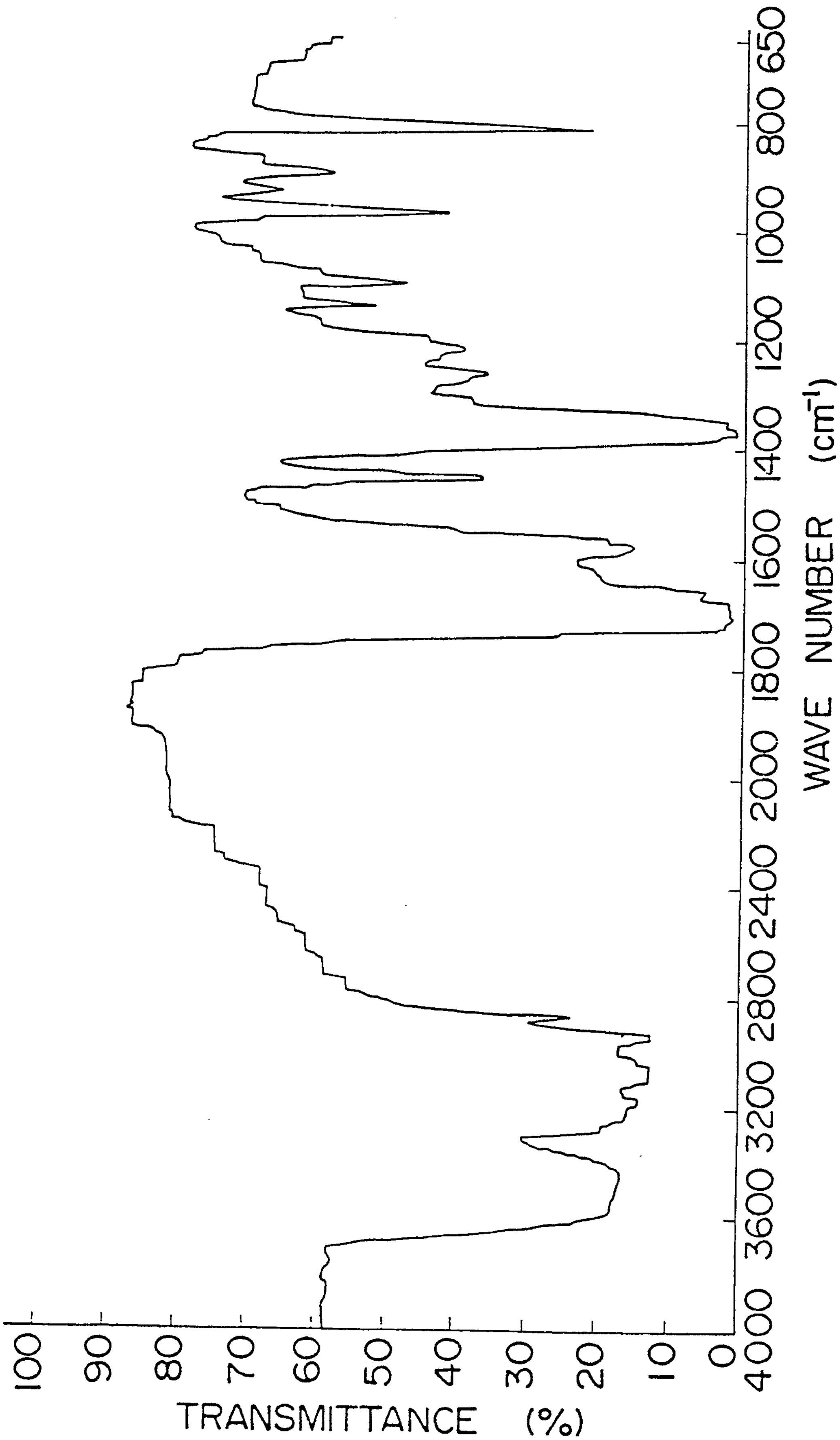

TRANSMITTANCE (%)
100 90 80 70 60 50 40 30 20 10 0
WAVE NUMBER (cm-1)
4000 3600 3200 2800 2400 2000 1800 1600 1400 1200 1000 800 650